(19) Europäisches Patentamt
European Patent Office
Office européen des brevets

(11) Veröffentlichungsnummer: **0 316 786 B1**

(12) **EUROPÄISCHE PATENTSCHRIFT**

(45) Veröffentlichungstag der Patentschrift: **27.11.91**

(51) Int. Cl.[5]: **C07C 255/12**, C07D 333/38

(21) Anmeldenummer: **88118791.8**

(22) Anmeldetag: **11.11.88**

# (54) Trialkylaminsalze von 1,1-Dicyano-2-hydroxy-3-chlorprop-1-en, sowie ein Verfahren zur Herstellung von 2-Amino-4-chlor-3-cyano-5-formylthiophen.

(30) Priorität: **17.11.87 DE 3738910**

(43) Veröffentlichungstag der Anmeldung:
**24.05.89 Patentblatt 89/21**

(45) Bekanntmachung des Hinweises auf die Patenterteilung:
**27.11.91 Patentblatt 91/48**

(84) Benannte Vertragsstaaten:
**CH DE FR GB IT LI**

(56) Entgegenhaltungen:
**EP-A- 0 193 885**

(73) Patentinhaber: **BASF Aktiengesellschaft**
**Carl-Bosch-Strasse 38**
**W-6700 Ludwigshafen(DE)**

(72) Erfinder: **Etzbach, Karl-Heinz, Dr.**
**Carl-Bosch-Ring 55**
**W-6710 Frankenthal(DE)**

**EP 0 316 786 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruoh ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft Trialkylaminsalze von 1,1-Dicyano-2-hydroxy-3-chlorprop-1-en, wobei das Trialkylamin jeweils 10 bis 24 Kohlenstoffatome aufweist, ein Verfahren zu seiner Herstellung, ausgehend von Malondinitril, Chloracetylchlorid und Trialkylamin, sowie ein Verfahren zur Herstellung von 2-Amino-4-chlor-3-cyano-5-formylthiophen, bei dem man das Trialkylaminsalz von 1,1-Dicyano-2-hydroxy-3-chlorprop-1-en mit Schwefelwasserstoff oder einem Sulfid cyclisiert, dann mit einem Gemisch aus N,N-Dimethylformamid und Phosphoroxychlorid umsetzt und das Reaktionsprodukt ohne Zwischenisolierung sauer verseift.

Aus der EP-A-193 885 ist die Herstellung von 2-Amino-4-chlor-3-cyano-5-formylthiophen bekannt. Man erhält diese Verbindung dort, wenn man aus Malondinitril, Chloracetylchlorid und Triethylamin intermediär das Triethylammoniumsalz von 1,1-Dicyano-2-hydroxy-3-chlorprop-1-en herstellt, dieses mit Ammoniumsulfid zu 2-Amino-3-cyano-4-hydroxythiophen cyclisiert, dann mit einem Gemisch aus N,N-Dimethylformamid und Phosphoroxychlorid umsetzt, das resultierende N,N-Dimethyl-N′-(4-chlor-3-cyano-5-formylthien-2-yl)-formamidin isoliert und nach seiner Isolierung mit Ameisensäure zum Zielprodukt verseift.

Es hat sich jedoch gezeigt, daß nach dem dort beschriebenen Verfahren nur eine unbefriedigende Ausbeute an 2-Amino-4-chlor-3-cyano-5-formylthiophen erhalten wird.

Aufgabe der vorliegenden Erfindung war es nun, neue Zwischenprodukte bereitzustellen, mittels derer die Herstellung von 2-Amino-4-chlor-3-cyano-5-formylthiophen in besserer Ausbeute gelingt.

Es wurden Trialkylaminsalze von 1,1-Dicyano-2-hydroxy-3-chlorprop-1-en gefunden, wobei das Trialkylamin jeweils 10 bis 24 Kohlenstoffatome aufweist.

Diese Salze, die die Formel I

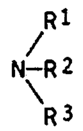 · 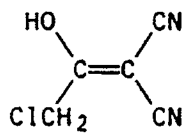 (I)

aufweisen, in der $R^1$, $R^2$ und $R^3$ gleich oder verschieden sind und unabhängig voneinander jeweils für $C_1$-$C_{22}$-Alkyl stehen, mit der Maßgabe, daß die Anzahl der Kohlenstoffatome 10 bis 24 beträgt, eignen sich in vorteilhafter Weise zur Herstellung von 2-Amino-4-chlor-3-cyano-5-formylthiophen der Formel II

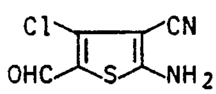 (II).

Geeignete Trialkylamine, die den erfindungsgemäßen Salzen zugrundeliegen, sind z.B. Tri-n-butylamin, Tri-n-pentylamin, Trihexylamin, Tris(2-ethylhexylamin), N,N-Dimethyl-N-(2-ethylhexyl)amin oder N-Methyl-N,N-bis(2-ethylhexyl)amin.

Bevorzugt sind solche Trialkylaminsalze von 1,1-Dicyano-2-hydroxy-3-chlorprop-1-en, bei denen das Trialkylamin jeweils 10 bis 17 Kohlenstoffatome und insbesondere 12 Kohlenstoffatome aufweist. Das Tri-n-butylaminsalz ist ganz besonders hervorzuheben.

Die erfindungsgemäßen Trialkylaminsalze von 1,1-Dicyano-2-hydroxy-3-chlorprop-1-en werden vorteilhaft erhalten, wenn man Malondinitril gleichzeitig mit Chloracetylchlorid und einem entsprechenden Trialkylamin umsetzt.

Die Umsetzung erfolgt bei einer Temperatur von -10 bis +100 °C, vorzugsweise 0 bis +20 °C in einem inerten organischen Lösungsmittel, z.B. in N,N-Dimethylformamid, N-Methylpyrrolid-2-on, Dioxan, Tetrahydrofuran, Methylenchlorid oder Methyl-tert-butylether. Bevorzugt ist die Verwendung von N,N-Dimethylformamid als Lösungsmittel. Das Molverhältnis von Malondinitril : Chloracetylchlorid : Trialkylamin beträgt dabei im allgemeinen 1 : 1 : 2 bis 1 : 1,5 : 3.

Zweckmäßig wird das neue Verfahren so durchgeführt, daß man eine Lösung von Malondinitril im organischen Lösungsmittel vorlegt und dann Chloracetylchlorid und Trialkylamin gleichzeitig, jedoch aus getrennten Zulaufgefäßen, unter Einhaltung der erfindungsgemäßen Temperatur zugibt. Nach einer Nachrührphase, die im allgemeinen 0,1 bis 1 Stunde erfordert, wird das Reaktionsgemisch auf kalte, verdünnte Salzsäure gegeben und mit Ether extrahiert. Die Etherphase wird dann getrocknet und vom Lösungsmittel

befreit.

Im Gegensatz zu Triethylamin können die den erfindungsgemäßen Salzen zugrundeliegenden Trialkylamine bei der Thiophenherstellung zurückgewonnen werden.

Es ist überraschend, daß die erfindungsgemäßen Trialkylaminsalze I in Wasser und verdünnten Mineralsäuren unlöslich sind und damit isoliert werden können. Sie entstehen in guter Ausbeute und hoher Reinheit und verfügen außerdem über eine hohe thermische Stabilität. Sie eignen sich besonders vorteilhaft als Zwischenprodukt für die Synthese von 2-Amino-4-chlor-3-cyano-5-formylthiophen oder auch von anderen Heterocyclen. Die letztgenannten Verbindungen sind wiederum wertvolle Zwischenprodukte für die Herstellung von Farbstoffen.

Es wurde nun weiterhin gefunden, daß die Herstellung von 2-Amino-4-chlor-3-cyano-5-formylthiophen durch Cyclisierung von 1,1-Dicyano-2-hydroxy-3-chlorprop-1-en mit Schwefelwasserstoff oder einem Sulfid und Umsetzung des resultierenden 2-Amino-3-cyano-4-hydroxythiophens mit einem Gemisch aus N,N-Dimethylformamid und Phosphoroxychlorid und saurer Verseifung des resultierenden N,N-Dimethyl-N′-(4-chlor-3-cyano-5-formylthien-2-yl)formamidins vorteilhaft gelingt, wenn man die Cyclisierung mit einem Trialkylaminsalz von 1,1-Dicyano-2-hydroxy-3-chlorprop-1-en durchführt, wobei das Trialkylamin jeweils 10 bis 24 Kohlenstoffatome aufweist, und die saure Verseifung von N,N-Dimethyl-N′-(4-chlor-3-cyano-5-formylthien-2-yl)formamidin ohne Zwischenisolierung vornimmt.

Die Herstellung von 2-Amino-3-cyano-4-hydroxythiophen der Formel III

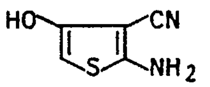 (III)

erfolgt durch Cyclisierung der erfindungsgemäßen Trialkylaminsalze I mit Schwefelwasserstoff oder einem Sulfid. Als Sulfide kommen dabei Alkali-, Erdalkali- oder Ammoniumsulfide oder -hydrogensulfide in Betracht, beispielsweise Natrium- oder Kaliumsulfid oder -hydrogensulfid, Calciumsulfid oder -hydrogensulfid oder Ammoniumsulfid oder -hydrogensulfid. Trialkylaminsalz I und Schwefelwasserstoff (oder Sulfid) werden dabei in der Regel im Molverhältnis 1 : 1 bis 1 : 2 eingesetzt.

Die Umsetzung findet vorzugsweise in Wasser bei einer Temperatur von 0 bis 80 °C statt. Nach beendeter Reaktion, die im allgemeinen 1 bis 4 Stunden in Anspruch nimmt, wird mit verdünnter Salzsäure versetzt und das 4-Hydroxythiophenderivat III abgetrennt, gewaschen und getrocknet.

Dann wird das 4-Hydroxythiophenderivat III durch Umsetzung mit einem Gemisch aus N,N-Dimethylformamid und Phosphoroxychlorid intermediär in das N,N-Dimethyl-N′-(4-chlor-3-cyano-5-formylthien-2-yl)-formamidin der Formel IV

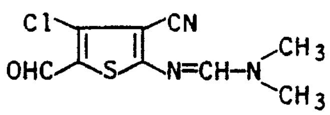 (IV)

übergeführt, das ohne Zwischenisolierung in saurem Milieu direkt zu 2-Amino-4-chlor-3-cyano-5-formylthiophen II verseift wird.

Zweckmäßig wird dabei das Hydroxythiophen III in N,N-Dimethylformamid gelöst und die Lösung anschließend bei einer Temperatur von 20 bis 80 °C mit Phosphoroxychlorid versetzt. Das Molverhältnis von Hydroxythiophen : N,N-Dimethylformamid : Phosphoroxychlorid beträgt dabei im allgemeinen 1 : 10 : 2 bis 1 : 20 : 3. Nach einer Nachrührphase von 2 bis 8 Stunden bei einer Temperatur von 20 bis 80 °C wird das intermediär gebildete Formamidin IV sauer verseift. Dies geschieht vorteilhaft dadurch, daß man das Reaktionsgemisch in Wasser gibt. Nach beendeter Zugabe, die stark exotherm ist, wird im allgemeinen 1 bis 4 Stunden bei einer Temperatur von 80 bis 100 °C nachgerührt. Das Thiophenderivat der Formel II fällt dabei aus und wird dann vom Reaktionsgemisch abgetrennt, gewaschen und getrocknet.

Das neue Verfahren liefert erheblich höhere Ausbeuten als die aus der EP-A-193 885 bekannte Methode. Außerdem fällt die Zwischenisolierung des Formamidins IV weg.

Wie bereits ausgeführt, ist 2-Amino-4-chlor-3-cyano-5-formylthiophen ein wertvolles Zwischenprodukt für die Synthese von Azofarbstoffen.

Die folgenden Beispiele sollen die Erfindung näher erläutern.

Beispiel 1

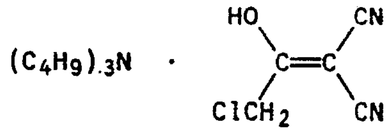

Zu einer Lösung von 33 g Malondinitril in 100 ml N,N-Dimethylformamid wurden bei einer Temperatur von 0 °C gleichzeitig 56,5 g Chloracetylchlorid und 185 g Tri-n-butylamin aus getrennten Zulaufgefäßen zugegeben. Die Mischung wurde nach beendeter Zugabe noch 15 Minuten bei 0 °C nachgerührt und dann auf 500 ml eiskalte, verdünnte Salzsäure gegeben. Das Gemisch wurde dreimal mit Ether extrahiert und die vereinigten organischen Phasen mit Wasser gewaschen und über Natriumsulfat getrocknet. Nach Abziehen des Ethers unter vermindertem Druck erhielt man 123 g (75 % d. Th.) des Tri-n-butylaminsalzes von 1,1-Dicyano-2-hydroxy-3-chlorprop-1-en als orangefarbenes Öl. IR (Film): 2963, 2937, 2876 (C-H), 2203, 2182 (C≡N), 1571 $cm^{-1}$ (C=O) $C_{17}H_{30}ClN_3O$ (327,5)
Ber.: C 62,27 H 9,22 Cl 10,81 N 12,81 O 4,88
Gef.: C 61,8 H 9,1 Cl 11,1 N 12,9 O 5,3
In analoger Weise werden die Salze von N,N-Dimethyl-N-(2-ethylhexyl)amin, Tris(2-ethylhexyl)amin und Tri-n-pentylamin erhalten.

Beispiel 2 (Vergleich)

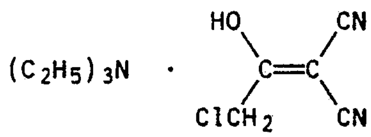

Analog Beispiel 1 wurden 33 g Malondinitril, 56,5 g Chloracetylchlorid und 101 g Triethylamin in 100 ml N,N-Dimethylformamid umgesetzt. Als man die Mischung auf verdünnte Salzsäure gab, blieb das entstandene Triethylaminsalz in Lösung und konnte nicht isoliert werden.

Beispiel 3

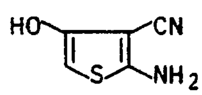

Ein Gemisch aus 9,5 g Tri-n-butylaminsalz aus Beispiel 1, 2,9 g Natriumsulfid (60 gew.%ig) und 100 ml Wasser wurde 4 Stunden bei Raumtemperatur gerührt und dann mit verdünnter Salzsäure angesäuert. Der resultierende Niederschlag wurde abgesaugt, mit Wasser gewaschen und getrocknet. Man erhielt 2,5 g (89 % d. Th.) 2-Amino-3-cyano-4-hydroxythiophen.
Schmelzpunkt >300 °C.

Beispiel 4

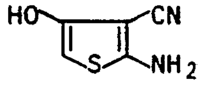

Zu einer Lösung von 231 g Malondinitril in 350 ml N,N-Dimethylformamid wurden bei einer Temperatur von 0°C gleichzeitig 396 g Chloracetylchlorid und 1295 g Tri-n-butylamin aus getrennten Zulaufgefäßen zugegeben. Nach beendeter Zugabe rührte man die Mischung noch 15 Minuten bei 0°C nach und gab sie dann in ein Gemisch aus 455 g 60 gew.%igem Natriumsulfid, 1000 g Eis und 1000 ml Wasser. Man rührte 3 Stunden nach, saugte das Produkt ab, wusch es mit Wasser, dann mit verdünnter Salzsäure und dann nochmals mit Wasser und trocknete es.

Man erhielt 425 g (87 % d. Th.) 2-Amino-3-cyano-4-hydroxythiophen. Schmelzpunkt > 300°C.

Die Beispiele 5 bis 7 wurden analog Beispiel 4 durchgeführt, jedoch wurde der Reaktionsansatz jeweils auf ein Siebtel reduziert, und anstelle von Tri-n-butylamin wurden andere Amine verwendet.

Beispiel 5

Verwendetes Amin: 157 g N,N-Dimethyl-N-(2-ethylhexyl)amin
Ausbeute an 2-Amino-3-cyano-4-hydroxythiophen: 58 g (83 % d.Th.)

Beispiel 6

Verwendetes Amin: 354 g Tris(2-ethylhexyl)amin
Ausbeute an 2-Amino-3-cyano-4-hydroxythiophen: 56,3 g (80 % d.Th.)

Beispiel 7

Verwendetes Amin: 227 g Tri-n-pentylamin
Ausbeute an 2-Amino-3-cyano-4-hydroxythiophen: 58,9 g (84 % d.Th.)

Beispiel 8

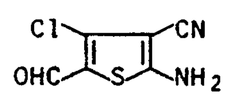

280 g 2-Amino-3-cyano-4-hydroxythiophen wurden in 1800 ml N,N-Dimethylformamid gelöst, dann gab man innerhalb von 30 Minuten 612 g Phosphoroxychlorid zu, dabei wurde die Temperatur durch Kühlung mit Wasser bei 70 bis 80°C gehalten. Nach beendeter Zugabe wurde die Lösung noch 2 Stunden bei 80°C nachgerührt und dann in 1800 ml Wasser gegeben. Hierbei erhitzte sich die Mischung unter Sieden auf ca. 110°C. Nach Abklingen der exothermen Reaktion erhitzte man das Gemisch noch 1 Stunde zum Sieden. Das Zielprodukt fiel hierbei aus und wurde nach dem Abkühlen der Mischung auf Raumtemperatur abgesaugt, mit Wasser gewaschen und getrocknet. Man erhielt 317 g (85 % d. Th.) 2-Amino-4-chlor-3-cyano-5-formylthiophen.
Schmelzpunkt 270°C.

## Patentansprüche

1. Trialkylaminsalze von 1,1-Dicyano-2-hydroxy-3-chlorprop-1-en, wobei das Trialkylamin jeweils 10 bis 24 Kohlenstoffatome aufweist.

2. Verfahren zur Herstellung der Trialkylaminsalze von 1,1-Dicyano-2-hydroxy-3-chlorprop-1-en gemäß Anspruch 1, dadurch gekennzeichnet, daß man Malondinitril gleichzeitig mit Chloracetylchlorid und Trialkylamin umsetzt.

3. Verfahren zur Herstellung von 2-Amino-4-chlor-3-cyano-5-formylthiophen durch Cyclisierung von 1,1-Dicyano-2-hydroxy-3-chlorprop-1-en mit Schwefelwasserstoff oder einem Sulfid und Umsetzung des resultierenden 2-Amino-3-cyano-4-hydroxythiophens mit einem Gemisch aus N,N-Dimethylformamid und Phosphoroxychlorid und saurer Verseifung des resultierenden N,N-Dimethyl-N'-(4-chlor-3-cyano-5-formylthien-2-yl)formamidins, dadurch gekennzeichnet, daß man die Cyclisierung mit einem Trialkylaminsalz von 1,1-Dicyano-2-hydroxy-3-chlorprop-1-en durchführt, wobei das Trialkylamin jeweils 10 bis 24 Kohlenstoffatome aufweist, und die saure Verseifung von N,N-Dimethyl-N′-(4-chlor-3-cyano-5-formylthien-2-yl)formamidin ohne Zwischenisolierung vornimmt.

## Claims

1. A trialkylamine salt of 1,1-dicyano-2-hydroxy-3-chloroprop-1-ene wherein the trialkylamine has from 10 to 24 carbon atoms.

2. A process for preparing a trialkylamine salt of 1,1-dicyano-2-hydroxy-3-chloroprop-1-ene as claimed in claim 1, which comprises reacting malodinitrile simultaneously with chloroacetyl chloride and a trialkylamine.

3. A process for preparing 2-amino-4-chloro-3-cyano-5-formylthiophene by cyclizing 1,1-dicyano-2-hydroxy-3-chloroprop-1-ene with hydrogen sulfide or another sulfide and reacting the resulting 2-amino-3-cyano-4-hydroxythiophene with a mixture of N,N-dimethylformamide and phosphoryl chloride and subjecting the resulting N,N-dimethyl-N'-(4-chloro-3-cyano-5-formylthien-2-yl)formamidine to acid hydrolysis, which comprises effecting the cyclization with a trialkylamine salt of 1,1-dicyano-2-hydroxy-3-chloroprop-1-ene where the trialkylamine salt has from 10 to 24 carbon atoms and conducting the acid hydrolysis of N,N-dimethyl-N'-(4-chloro-3-cyano-5-formylthien-2-yl)formamidine without intermediate isolation.

## Revendications

1. Sels de trialkylamine et de 1,1-dicyano-2-hydroxy-3-chloroprop-1-ène, la trialkylamine renfermant chaque fois de 10 à 24 atomes de carbone.

2. Procédé de préparation de sels de trialkylamine et de 1,1-dicyano-2-hydroxy-3-chloroprop-1-ène selon la revendication 1, caractérisé en ce qu'on fait réagir du malodinitrile simultanément avec du chlorure de chloracetyle et une trialkylamine.

3. Procédé de préparation de 2-amino-4-chloro-3-cyano-5-formylthiophène par cyclisation de 1,1-dicyano-2-hydroxy-3-chloroprop-1-ène avec de l'acide sulfhydrique ou avec un sulfure, mise en réaction du 2-amino-3-cyano-4-hydroxythiophène résultant avec un mélange de N,N-dimethylformamide et d'oxychlorure de phosphore et saponification acide de la N,N-dimethyl-N'-(4-chloro-3-cyano-5-formylthiène-2-yl)-formamidine résultante, caractérisé en ce qu'on effectué la cyclisation avec un sel de trialkylamine et de 1,1-dicyano-2-hydroxy-3-chloroprop-1-ène, la trialkylamine renfermant chaque fois de 10 à 24 atomes de carbone, et en ce qu'on procède à la saponification acide de la N,N-dimethyl-N'-(4-chloro-3-cyano-5-formylthiène-2-yl)-formamidine sans isolement intermédiaire.